# FASCICULE DE BREVET EUROPEEN

(11) **EP 2 614 850 B1**
(45) Date de publication et mention de la délivrance du brevet: **16.04.2014**
(21) Numéro de dépôt: 13305015.3
(22) Date de dépôt: 09.01.2013
(51) Int. Cl.: A61M 15/00, A61F 11/00, A61M 11/06, A61M 15/08

(54) **Dispositif pour appliquer un stimulus de pression pneumatique dans les fosses nasales et dans la trompe auditive au moment de la déglution**
Gerät zum Ausüben eines pneumatischen Druckstimulus auf die Nasenhöhlen und den Gehörgang beim Schlucken
Device for applying a pneumatic pressure stimulus to the nasal cavities and to the Eustachian tube at the time of the swallowing

(30) Priorité: 11.01.2012 FR 1250281
(43) Date de publication de la demande: 17.07.2013
(73) Titulaire: LA DIFFUSION TECHNIQUE FRANCAISE, 42000 Saint Etienne (FR)
(72) Inventeur: Massardier, Michel, 42000 Saint-Etienne (FR); Chantrel, Gilles, 42100 Saint-Etienne (FR); Esteve, Dominique, 04800 Greoux les Bains (FR)
(74) Mandataire: Dupuis, François

(56) Documents cités:
- DE-A1- 3 617 400
- FR-A1- 2 674 756
- FR-A1- 2 824 479

## Description

### DOMAINE TECHNIQUE

La présente invention a trait à un dispositif pour appliquer un stimulus de pression dans les fosses nasales et pour injecter une dose d'air dans la trompe auditive au moment de la déglutition et plus particulièrement à des moyens permettant de s'assurer que la déglutition est correctement effectuée lors du stimulus de pression.

### ART ANTERIEUR

La trompe auditive ou trompe d'Eustache est un conduit osseux et fibro-cartilagineux reliant la paroi antérieure de l'oreille moyenne au rhinopharynx, c'est-à-dire la partie de la gorge qui se situe au dessus du voile du palais, en arrière du nez. Cette trompe auditive a plusieurs fonctions, une fonction mécanique empêchant par sa fermeture l'introduction d'agents pathogènes et de sécrétions nasales dans l'oreille moyenne, une fonction de clairance muco-cilaire dans sa partie basse permettant d'évacuer les corps gênants de l'oreille moyenne et une fonction équipressive permettant d'égaliser la pression des deux côtés du tympan pour éviter sa rupture en cas de grande différence de pression entre le milieu extérieur et l'oreille moyenne.

La trompe auditive est fermée au repos et s'ouvre pendant une fraction de seconde lors de la déglutition notamment. La déglutition se traduit par un abaissement du voile, une rétro-pulsion de la langue, une élévation de l'os hyoïde. Lors de la phase isthmique, c'est-à-dire lorsque le bol alimentaire s'apprête à franchir l'isthme du gosier, la contraction de vélo-pharynx est maximale, les muscles tenseurs, élévateur du voile, et le pharyngo-staphylin se contractant de façon synergique. La protraction des mâchoires augmente le diamètre antéro-postérieur du pharynx et, lorsqu'elle est accompagnée d'une contraction de l'isthme du gosier, elle provoque l'ouverture de la trompe.

Il est reconnu qu'un dysfonctionnement de la trompe auditive peut conduire à différentes pathologies dont des infections récidivantes, des otites à répétition, des otites séreuses ou séro-muqueuses, ou une hypoacousie.

De plus, la majorité des dysfonctionnements de la trompe auditive, également dénommés dysfonctionnement tubaires, sont associés à des défauts primitifs ou secondaires (Takahasi H, Hayashi M, Sato H, Honjo I. « Primary deficits in eustachian tube function in patients with otitis media with effusion » Arch Otolaryngol Head Neck Surg. 1989 ; 1145 ; 581-584 *-* Iwano T, Kinoshita E, Doi T, Ushiro K, Kumazawa T. « Otitis media with effusion and eustachian tube dysfunction in adults and children. » Acta Otolaryngol Suppl. 1993 ; 500 ; 66-9) et que la trompe, physiologiquement fermée au repos, s'ouvre essentiellement par l'action synergique de muscles dont les principaux sont les muscles péristaphylins.

Ainsi, pour corriger les dysfonctionnements de la trompe auditive, il a été proposé des exercices de rééducation tubaire notamment par Jacobs A. dans une thèse intitulée « La kinésithérapie de la trompe d'Eustache. » (Thèse de médecine Nancy, 1981, 146 p*.).*

Toutefois, les exercices de kinésithérapie de la trompe auditive sont particulièrement difficiles à réaliser notamment pour des enfants qui constituent la grande majorité des patients atteints de dysfonctionnement de la trompe auditive de sorte que la kinésithérapie est rarement prescrite.

Afin de palier les inconvénients de la kinésithérapie de la trompe auditive, il est connu de pratiquer une rééducation de la trompe auditive en l'entraînant dans une réaction réflexe à un stimulus de pression au moyen d'un dispositif adapté. Ainsi, le Demandeur a lui-même développé un tel dispositif qui fait l'objet d'une exploitation.

Un tel dispositif qui permet d'injecter une dose d'air dans la trompe auditive, au moment de la déglutition, c'est-à-dire fournissant une quantité d'air définie, dès l'apparition de la fermeture du voile du palais, qui est à l'origine des mécanismes musculaires aboutissant à l'ouverture de la trompe auditive, est notamment décrit dans le brevet français FR 2 824 479 lui appartenant.

Ce dispositif pour appliquer un stimulus de pression pneumatique dans les fosses nasales et dans la trompe auditive au moment de la déglutition comprend notamment une pompe débitant en continu un flux d'air dans un circuit d'alimentation équipé d'une soupape de décharge à fuite calibrée, un réservoir d'air alimenté par un circuit dérivé du circuit d'alimentation et un circuit de fluide de détection et de commande allant à un embout narinaire faisant partie d'un boîtier équipé d'un ajutage de fuite, ledit circuit communiquant avec le réservoir et étant équipé de moyens d'obturation à membrane élastiquement déformable.

L'utilisation du dispositif s'effectue en sélectionnant une surpression déterminée (généralement comprise entre 15 et 80 mbars), en prenant une petite gorgée d'eau puis en fermant la bouche et en serrant les dents, la respiration s'effectuant alors calmement par le nez, puis en plaçant l'embout narinaire de manière étanche. Le patient arrête alors de respirer, bouche la prise d'air et avale la petite gorgée d'eau en conservant les dents serrées, la prise d'air du dispositif ne devant être débouchée qu'au bout de 2 ou 3 secondes. L'opération est répétée autant de fois que nécessaire, usuellement une quinzaine de fois.

Toutefois, l'utilisation de ce dispositif n'est efficace que si la déglutition est correctement effectuée.

Il existe donc un besoin pour un dispositif pour la rééducation de la trompe auditive par application d'un stimulus de pression dans le rhinopharynx d'un sujet dans lequel la bonne exécution du traitement peut être mesurée.

### EXPOSE DE L'INVENTION

L'un des buts de l'invention est donc de remédier à ces inconvénients en proposant un dispositif optimisé pour la rééducation de la trompe auditive par application d'un stimulus de pression dans le rhinopharynx d'un sujet de conception simple, peu onéreuse et permettant de mesurer l'efficacité du traitement.

A cet effet, et conformément à l'invention, il est proposé un dispositif pour appliquer de façon automatique une phase de surpression pneumatique, de valeur comprise entre 5 et 100 hPa, dans la trompe auditive et/ou dans les fosses nasales d'un sujet humain à travers au moins une narine au moment de la déglutition, ledit dispositif comprenant au moins un générateur de gaz, un réservoir de gaz et un circuit de fluide de détection et de commande communiquant avec le réservoir et le générateur de gaz et allant à un embout étanche au niveau des narines faisant partie d'une pièce nasale équipée d'une buse de commande ; ledit dispositif est remarquable en ce qu'il comporte au moins un moyen de mesure d'au moins une valeur caractéristique d'un écoulement de gaz lors de la déglutition, un moyen de comparaison automatique de ladite valeur caractéristique avec au moins une valeur prédéterminée, et un moyen d'information du sujet humain apte à indiquer si la déglutition est correctement effectuée.

On comprend bien que, contrairement aux dispositifs de l'art antérieur, le dispositif permet de s'assurer que la déglutition est correctement effectuée de telle manière qu'un nombre de déglutitions correctement effectuées, pour une surpression déterminée, est réalisée lors de la séance de traitement, la rendant plus efficace.

Selon une première variante d'exécution, le dispositif comporte au moins un moyen de mesure de la variation de débit et/ou de la durée de la variation de débit lors de la déglutition et un moyen de comparaison automatique de la valeur de la variation de débit et/ou de la durée de la variation de débit par rapport à au moins une valeur de variation de débit et/ou à au moins une durée de variation de débit prédéterminée.

Alternativement, selon une autre variante d'exécution, le dispositif comporte au moins un moyen de mesure de la surpression et/ou de la durée de la surpression lors de la déglutition et un moyen de comparaison automatique de la surpression et/ou de la durée de la surpression par rapport à au moins une valeur de surpression et/ou à au moins une durée de surpression prédéterminée

De préférence, le moyen d'information indique l'écart entre la valeur de surpression et/ou la durée de surpression mesurée avec au moins une valeur de surpression et/ou au moins une durée de surpression prédéterminée.

Par ailleurs, ledit moyen de comparaison automatique compare la valeur de surpression et/ou la durée de surpression mesurée avec au moins une valeur de surpression et/ou à au moins une durée de surpression prédéterminée sur tout ou partie d'au moins l'une des trois phases de surpression appliquée dans le rhinopharynx lors de la déglutition, une phase de montée de la surpression, une phase dite plateau de la surpression pendant laquelle la surpression est sensiblement constante pendant une durée déterminée, et une phase de descente de ladite surpression.

La durée prédéterminée de la montée en surpression est comprise entre 0,05 et 0,8 seconde.

La durée prédéterminée de la phase plateau de surpression est comprise entre 0,5 et 5 secondes.

La durée de la phase de descente est comprise entre 0,05 et 0,8 seconde.

La valeur de surpression prédéterminée consiste en un pourcentage de la valeur de surpression mesurée à un instant prédéterminé de la phase plateau.

De manière avantageuse, le moyen d'information comporte des moyens d'alerte visuelle et/ou sonore. Ces moyens d'alerte visuelle consistent dans un écran sur lequel sont aptes à s'afficher des messages.

De plus, le moyen de comparaison comporte au moins un moyen de pilotage d'au moins un moyen de commande apte à faire varier la valeur de la surpression mesurée. Ledit moyen de commande consiste de préférence dans une électrovanne.

Avantageusement, le dispositif comporte des moyens de transfert des valeurs de surpression mesurées et/ou des valeurs des durées de surpression mesurées et/ou de la valeur de surpression prédéterminée et/ou de la valeur de la durée de surpression déterminée et/ou des écarts déterminés par le moyen de comparaison.

De préférence, le dispositif comporte une soupape dite de sécurité connectée au tuyau flexible afin de limiter la pression dans ledit tuyau flexible en dessous d'une pression déterminée, préférentiellement en dessous de 100 mbars.

### DESCRIPTION SOMMAIRE DES FIGURES

D'autres avantages et caractéristiques ressortiront mieux de la description qui va suivre, de plusieurs variantes d'exécution, données à titre d'exemples non limitatifs, d'un dispositif pour la rééducation de la trompe auditive, en référence aux dessins annexés sur lesquels :
- la figure 1 est une vue en perspective du dispositif pour la rééducation de la trompe auditive,
- la figure 2 est une représentation schématique du dispositif pour la rééducation de la trompe auditive,
- la figure 3 est une représentation graphique de la courbe de la surpression mesurée dans la trompe auditive en fonction du temps lors de la déglutition
- la figure 4 est une représentation schématique d'une variante d'exécution du dispositif pour la rééducation de la trompe auditive
- les figures 5 et 6 sont des représentations schématiques de la variante d'exécution du dispositif au cours des différentes étapes de fonctionnement dudit dispositif.

### DESCRIPTION DETAILLEE DE L'INVENTION

On décrira ci-après un dispositif pour la rééducation de la trompe auditive par application d'un stimulus pneumatique dans les fosses nasales et dans la trompe auditive au moment de la déglutition ; toutefois, il est bien évident que le dispositif suivant l'invention pourra trouver d'autres applications telles que la « Tubomanométrie » par exemple sans sortir du cadre de l'invention.

Par souci de clarté, dans la suite de la description, les mêmes éléments ont été désignés par les mêmes références aux différentes figures. De plus, les diverses vues en coupe ne sont pas nécessairement tracées à l'échelle et les dimensions des éléments peuvent avoir été exagérées pour faciliter la compréhension de l'invention.

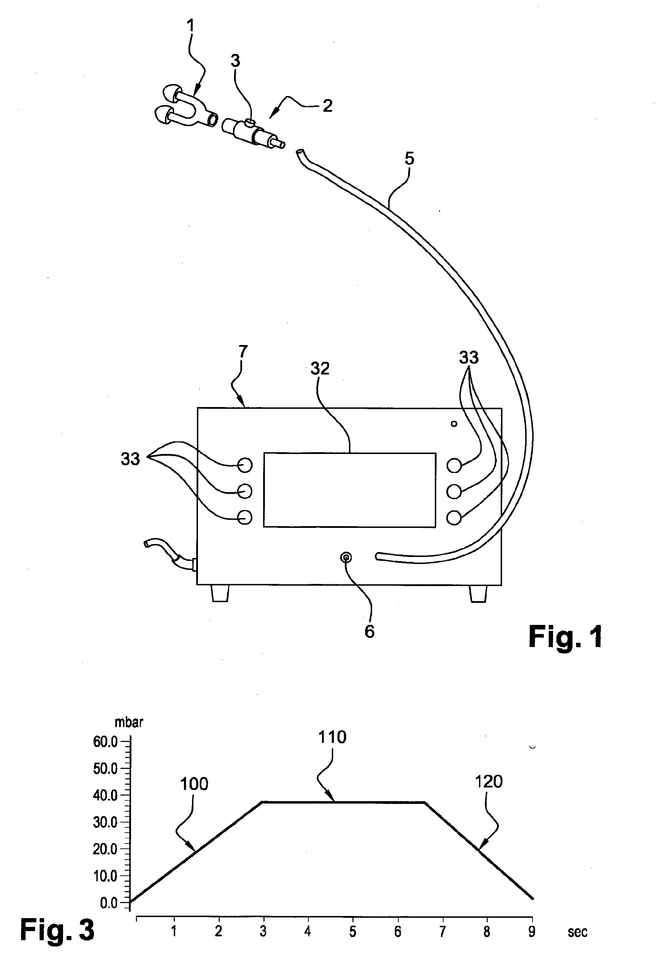

En référence à la figure 1, le dispositif comporte un embout narinaire (1) raccordé à une pièce dite nasale (2) cylindrique comprenant une buse de commande (3) et une buse d'arrivée d'air (4) et un tuyau (5) flexible raccordant la buse d'arrivée d'air (4) à une buse de sortie d'air (6) d'un boîtier (7).

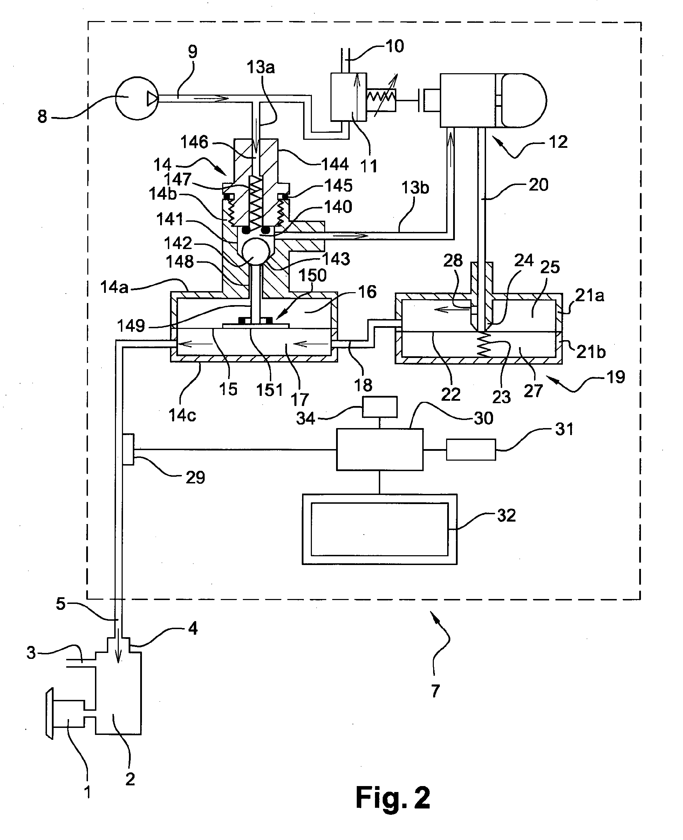

En référence aux figures 1 et 2, le boîtier (7) comporte, de la même manière que dans la demande de brevet français FR 2 824 479, un générateur de gaz (8) tel qu'une pompe pneumatique électrique débitant, dans un conduit d'alimentation (9), un flux continu d'air sous une pression déterminée par un ajutage calibré (10) formé par l'orifice de sortie d'une soupape de décharge (11) à tarage réglable. Le boîtier (7) comporte, par ailleurs, un réservoir de gaz (12) connecté au circuit d'alimentation (9) par une branche amont (13a) et une branche aval (13b) d'un circuit dérivé, les branches (13a) et (13b) du circuit dérivé étant raccordées l'une à l'autre par une vanne pneumatique (14).

Ladite vanne pneumatique (14) est composée d'un corps en deux parties, une première partie (14a) en forme de boîte ouverte de laquelle fait saillie un col (14b) et une seconde partie (14c) en forme de boîte ouverte. Les parties (14a) et (14c) sont solidarisées l'une à l'autre, de manière étanche, en enserrant entre elles une membrane élastiquement déformable (15). Cette membrane (15) forme, entre les deux boîtes (14a,14c), une chambre d'équilibrage (16) et une chambre de commande (17). Cette dernière comporte un orifice formant la sortie d'air (6) et est reliée par un conduit (18) à un détecteur de déglutition (19).

Le col (14b) de la vanne pneumatique (14) comprend un logement axial (140) ménagé dans une douille métallique (141) et recevant une bille métallique (142), la branche (13b) du circuit dérivé débouchant dans le logement axial (140). L'extrémité inférieure du logement axial (140) comporte un siège (143) apte à recevoir la bille (142) lorsque cette dernière est en position de repos et l'extrémité supérieure dudit logement axial (140) est fermée par un bouchon (144) vissé sur le col (14b) en plaquant un joint torique (145) positionné à l'extrémité supérieure dudit col (14b). Ledit bouchon (144) comporte un canal d'entrée (146) communiquant avec la branche amont (13a) du circuit dérivé et dont la partie inférieure est élargie pour recevoir un ressort hélicoïdal (147) qui tend à repousser la bille (142) dans le siège (143). De plus, l'extrémité inférieure du siège (143) comporte un alésage centrale (148) débouchant dans la chambre d'équilibrage (16) et dans lequel s'étend d'une tige (149) d'un poussoir (150) dont la tête (151) prend appui sur la membrane (15) de la vanne pneumatique (14).

Ledit détecteur de déglutition (19) est connecté au réservoir (12) par une conduite (20) et est composé de deux parties (21a,21b) qui sont assemblées de manière étanche de part et d'autre d'une membrane élastiquement déformable (22) qui, en position de repos et avec l'aide d'un ressort (23), obture une buse (24) connectée à la conduite (20). Cette membrane (22) divise le détecteur de déglutition (19) en une chambre de commande (25) reliée à la chambre d'équilibrage (16) de la vanne pneumatique (14) par une conduite (26) et en une chambre d'équilibrage (27). La buse (24) comporte un ajutage calibré complémentaire (28) qui laisse passer un débit d'air formant flux de détection et de commande.

Ainsi, la pièce nasale (2) équipée d'une buse de commande (3) forme avec le tuyau flexible (5), la chambre de commande (17), la conduite (26), la chambre de commande (25) et la buse (24), le circuit de détection et de commande en mesure de transmettre:
- à la pièce nasale (2), le flux de détection et de commande débité par l'ajutage (28),
- aux chambres (17) et (25), la montée en pression du flux précité, consécutivement à la mise en étanchéité de la sphère ORL par la déglutition et à la fermeture de buse de commande (3),
- et à l'embout narinaire (1), la dose d'air en surpression libérée par le réservoir (12).

Par ailleurs, en référence à la figure 2, le dispositif comporte un capteur de pression (29) connecté à une unité centrale de traitement (30) dite CPU selon l'acronyme anglo-saxon « Central Processing Unit » incluant de manière classique une horloge ; ledit capteur de pression (29) étant positionné dans le tuyau (5) flexible raccordant la buse d'arrivée d'air (4) de la pièce nasale (2) à la buse de sortie d'air (6) du boîtier (7). Ce capteur de pression (29) permet de mesurer de manière continue dans le temps la valeur de la surpression et/ou de la durée de la surpression lors la déglutition comme il sera détaillé plus loin. L'unité centrale de traitement (30) est connectée à une unité de mémoire (31) et à des moyens d'affichage (32) tel qu'un écran LCD par exemple.

L'unité de mémoire (31) pourra consister dans toute unité de mémoire bien connue de l'homme du métier telle qu'une DRAM selon l'acronyme anglo-saxon « Dynamic Random Access Memory », une SDRAM selon l'acronyme anglo-saxon « Synchronous Dynamic Random Access Memory », une mémoire flash ou similaire.

De plus, il est bien évident que l'unité centrale de traitement (30) pourra ne pas comporter d'horloge interne. Dans cette hypothèse, le dispositif comportera une horloge externe connectée à l'unité centrale de traitement (30).

Ladite unité centrale de traitement (30) comporte, par ailleurs, des moyens de comparaison de la valeur de la surpression et/ou de la durée de la surpression pour au moins l'une des phases par rapport à des valeurs de surpression et/ou des durées de surpression prédéterminées afin de s'assurer que la déglutition est correctement effectuée lors du stimulus de pression, la courbe de la surpression comportant trois phases lors de la déglutition, en référence à la figure 3, une phase de montée de la surpression (100), une phase dite plateau (110) de la surpression pendant laquelle la surpression est sensiblement constante pendant une durée déterminée, et une phase de descente (120) de ladite surpression.

Ces moyens de comparaison consistent dans un programme d'ordinateur incluant des algorithmes enregistrés soit dans l'unité centrale de traitement (30) soit dans l'unité de mémoire (31). On entend par algorithme une suite finie d'opérations élémentaires constituant un schéma de calculs ou de résolutions d'un problème.

Lesdits moyens de comparaison comportent des premiers moyens de comparaison de la durée de la phase de montée (100) de la surpression mesurée par le capteur (29) avec une durée prédéterminée enregistrée dans une unité de mémoire. La durée prédéterminée de la montée en surpression est comprise entre 0,05 et 0,8 seconde. Ainsi, l'algorithme formant les premiers moyens de comparaison détecte la phase de montée (100) de la courbe de pression mesurée en fonction du temps, puis il détermine la durée de cette phase de montée (100) à partir de la courbe de mesure de la pression. Cette durée mesurée de la phase de montée (100) est comparée avec une plage de valeurs encadrée par deux valeurs seuils prédéterminées enregistrées dans l'unité de mémoire (31).

Si la durée mesurée de la phase de montée (100) se situe en dehors de la plage de valeurs encadrées par les valeurs seuils, l'unité centrale de traitement (30) génère un message indiquant un échec de la déglutition qui s'affiche alors sur l'écran (32).

Si la durée mesurée de la phase de montée (100) est comprise dans la plage de valeurs encadrées par les valeurs seuils, l'unité centrale de traitement (30) exécute éventuellement un second algorithme correspondant à des seconds moyens de comparaison.

Ces seconds moyens de comparaison comparent la durée de la phase plateau (110) de surpression avec une durée prédéterminée enregistrée dans l'unité de mémoire (31). La durée prédéterminée de la phase plateau (110) de surpression est comprise entre 0,5 et 5 secondes. Ainsi, l'algorithme formant les seconds moyens de comparaison détecte la phase plateau (110) de la courbe de pression mesurée en fonction du temps, puis il détermine la durée de cette phase plateau (110) à partir de la courbe de mesure de la pression. Cette durée mesurée de la phase plateau (110) est comparée avec une plage de valeur encadrée par deux valeurs seuils prédéterminées enregistrées dans l'unité de mémoire (31).

Si la durée mesurée de la phase plateau (110) se situe en dehors de la plage de valeurs encadrée par les valeurs seuils, l'unité centrale de traitement (30) génère un message indiquant un échec de la déglutition qui s'affiche sur l'écran (32).

Par contre, si la durée mesurée de la phase plateau (110) est comprise dans la plage de valeurs encadrée par les valeurs seuils, l'unité centrale de traitement (30) exécute éventuellement un troisième algorithme correspondant à des troisièmes moyens de comparaison.

Ces troisièmes moyens de comparaison comparent la valeur de surpression mesurée au cours de la phase plateau (110) avec une plage de valeurs encadrée par deux valeurs seuils de surpression prédéterminées enregistrées dans l'unité de mémoire (31). Ces deux valeurs seuils de surpression prédéterminées consistent respectivement en un pourcentage de la valeur de surpression mesurée à un instant prédéterminé de la phase plateau (110). Ainsi, l'algorithme formant les troisièmes moyens de comparaison détecte la phase plateau (110) de la courbe de pression mesurée en fonction du temps, puis il détermine la valeur de la surpression au début de cette phase plateau (110) et la valeur de la surpression à la fin de la phase plateau (110). L'algorithme détermine ensuite la valeur seuil de surpression en fonction de la valeur de surpression mesurée au début de la phase plateau (110) puis il compare la valeur mesurée à la fin de la phase plateau (110) avec la plage de valeurs seuils préalablement calculée.

Si la valeur mesurée de la surpression à la fin de la phase plateau (110) se situe en dehors de la plage de valeurs, l'unité centrale de traitement (30) génère un message indiquant un échec de la déglutition qui s'affiche sur l'écran (32).

Il est bien évident que, de manière alternative, l'algorithme pourra exécuter l'un quelconque des moyens de comparaison précédemment décrits ou une combinaison quelconque de ces trois moyens de comparaison.

On notera que les messages d'échec de la déglutition correspondant aux seconds et troisièmes moyens de comparaison peuvent également correspondre à une perte d'étanchéité au niveau de l'embout narinaire (1). Le patient est alors invité à vérifier l'adaptation de l'embout aux narines et éventuellement à changer de taille d'embout. De plus, l'embout étanche peut être raccordé à une seule narine, l'autre narine étant alors obturée.

On observera que le dispositif pourra également comporter des moyens d'alerte sonore signalant un échec de la déglutition sans pour autant sortir du cadre de l'invention.

Par ailleurs, on notera que l'utilisateur pourra paramétrer notamment la valeur de surpression, usuellement entre 5 et 100 hPa, et/ou le nombre de déglutitions à réaliser et/ou les valeurs seuils de surpression et/ou de durées de surpression au moyen de boutons (33) de commande et de sélection positionnés de part et d'autre de l'écran (32), en référence à la figure 1.

De manière avantageuse, le dispositif comporte des moyens de transfert (34) des valeurs de surpression mesurées et/ou des valeurs des durées de surpression mesurées et/ou de la valeur de surpression prédéterminée et/ou de la valeur de la durée de surpression déterminée et/ou des écarts déterminés par le moyen de comparaison. Ces moyens de transfert consistent en un bus informatique permettant de se connecter sur un réseau et/ou en des moyens de communication sans fil tels que Bluetooth®, WI-FI®, Zigbee®, etc... ou similaire.

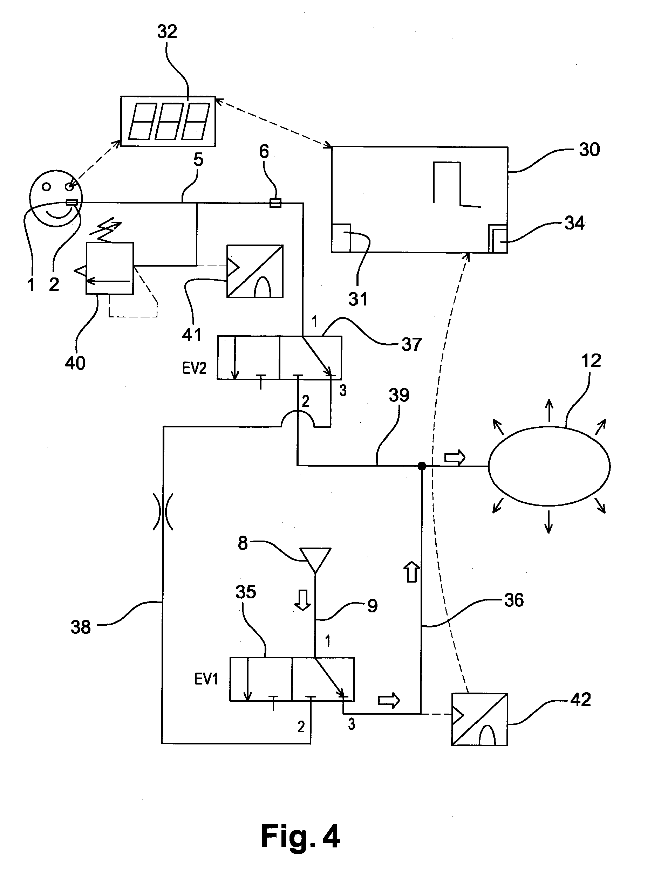

Selon une variante d'exécution du dispositif suivant l'invention, en référence à la figure 4, le dispositif comporte de la même manière que précédemment un embout narinaire (1) raccordé à une pièce dite nasale (2) cylindrique comprenant une buse de commande (3) et une buse d'arrivée d'air (4) et un tuyau (5) flexible raccordant la buse d'arrivée d'air (4) à une buse de sortie d'air (6) d'un boîtier (7), un générateur de gaz (8) tel qu'une pompe pneumatique électrique débitant, dans un conduit d'alimentation (9), un flux continu d'air sous une pression déterminée. Le conduit d'alimentation (9) est connecté à une première électrovanne (35) à trois voies, la première voie étant connectée audit conduit d'alimentation (9), la troisième voie étant connectée à un réservoir (12) par un conduit (36) et la seconde voie étant connectée à une voie d'une seconde électrovanne (37) par un conduit (38). Cette seconde électrovanne (37) comporte également trois voies, une première voie étant connectée au tuyau flexible (5) au travers d'une buse de sortie d'air (6), une troisième voie étant connectée par le conduit (38) à la seconde voie de la première électrovanne (35) et la seconde voie étant connectée au réservoir (12) par un conduit (39).

Le dispositif comporte par ailleurs une soupape (40) dite de sécurité connectée au tuyau flexible (5) afin de limiter la pression dans le tuyau flexible (5) en dessous de 100 mbars. En effet, lorsque la pression dans le tuyau flexible (5) atteint 100 mbars, la soupape (40) s'ouvre pour laisser s'échapper de l'air.

Par ailleurs, le dispositif comporte un premier capteur de pression (41) positionnés sur le tuyau flexible (5) et un second capteur de pression (42) positionné sur le conduit (36) alimentant le réservoir (12), lesdits capteurs de pression (41) et (42) étant connectés à une unité centrale de traitement (30) dite CPU selon l'acronyme anglo-saxon « Central Processing Unit » incluant de manière classique une horloge. L'unité centrale de traitement (30) est connectée à une unité de mémoire (31) et à des moyens d'affichage (32) tel qu'un écran LCD par exemple. Ladite unité centrale de traitement (30) comporte, par ailleurs, des moyens de comparaison de la valeur de la surpression et/ou de la durée de la surpression pour au moins l'une des phases par rapport à des valeurs de surpression et/ou des durées de surpression prédéterminées afin de s'assurer que la déglutition est correctement effectuée lors du stimulus de pression.

Le dispositif fonctionne ainsi de la manière suivante.

En référence à la figure 4, la première électrovanne (35) est en position dite de repos, dans laquelle la première voie est connectée à la troisième voie, alimentant ainsi le réservoir (12) qui se remplit, la seconde électrovanne (37) étant également en position dite de repos dans laquelle la première voie connectée au tuyau flexible (5) et la troisième voie sont connectées à la seconde voie de la première électrovanne (35).

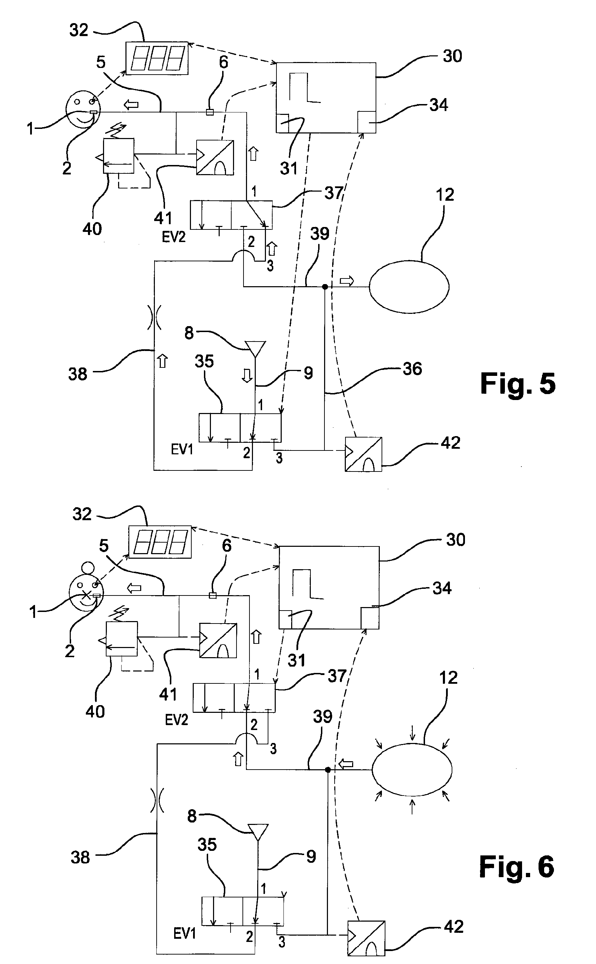

En référence à la figure 5, lorsqu'une pression déterminée est atteinte dans le réservoir (12), ladite pression étant mesurée par le second capteur de pression (42), l'unité centrale de traitement (30) actionne la première électrovanne (35) de sorte que la première voie soit désormais en communication avec la deuxième voie. De cette manière, le réservoir (12) n'est plus alimenté en air et la pompe (8) délivre un filet d'air, par le biais de la voie 1-3 de la seconde électrovanne (37), dans le nez du patient à travers l'embout narinaire (1) raccordé à la pièce nasale (2).

En référence à la figure 6, lorsque le patient déglutit, le voile de son palais se ferme et la pression dans le tuyau flexible (5) augmente. Le premier capteur de pression (41) mesure en continu la pression dans le tuyau flexible (5) et lorsque la pression mesurée est supérieure ou égale à une valeur prédéterminée, l'unité centrale de traitement (30) actionne la seconde électrovanne (37) de telle manière que la première voie soit en communication avec la seconde voie connectée au réservoir (12) au moyen du conduit (39). L'embout narinaire (1) est alors alimenté en air par le réservoir (12), par le biais de la voie 1-2 de la seconde électrovanne (37), et l'unité de centrale de traitement (30) contrôle la déglutition comme décrit précédemment. A la fin de la déglutition, ladite unité de traitement (30) actionne les deux électrovannes (35) et (37) de telle manière que la pompe (8) alimente à nouveau le réservoir (12) comme représenté sur la figure 4 et un nouveau cycle de déglutition peut recommencer.

Il va de soi que, la pression étant dépendante du débit d'air conformément au théorème de Bernoulli, au moins un des capteurs de pression (41,42) peut être substitué par un capteur de débit, l'unité de traitement (30) comportant alors un moyen de comparaison automatique de la valeur de la variation de débit (ou de la surpression) et/ou de la durée de la variation de débit (ou de surpression) par rapport à au moins une valeur de variation de débit (ou de surpression) et/ou à au moins une durée de variation de débit (ou de surpression) prédéterminée, sans pour autant sortir du cadre de l'invention.

Enfin, il est bien évident que les exemples que l'on vient de donner ne sont que des illustrations particulières en aucun cas limitatives quant aux domaines d'application de l'invention.

## Revendications

1. Dispositif pour appliquer de façon automatique une phase de surpression pneumatique, de valeur comprise entre 5 et 100 hPa, dans la trompe auditive et/ou dans les fosses nasales d'un sujet humain à travers au moins une narine au moment de la déglutition, ledit dispositif comprenant au moins un générateur de gaz (8), un réservoir de gaz (12) et un circuit de fluide de détection et de commande communiquant avec le réservoir (12) et le générateur de gaz (8) et allant à un embout (1) étanche au niveau des narines faisant partie d'une pièce nasale (2) équipée d'une buse de commande (3), ***caractérisé* en ce qu'il** comporte au moins un moyen de mesure (29,41) d'au moins une valeur caractéristique d'un écoulement de gaz lors de la déglutition, un moyen de comparaison automatique (30,31) de ladite valeur caractéristique avec au moins une valeur prédéterminée, et un moyen d'information (32) du sujet humain apte à indiquer si la déglutition est correctement effectuée.

2. Dispositif selon la revendication 1 ***caractérisé* en ce qu'il** comporte au moins un moyen de mesure de la variation de débit et/ou de la durée de la variation de débit lors de la déglutition et un moyen de comparaison automatique de la valeur de la variation de débit et/ou de la durée de la variation de débit par rapport à au moins une valeur de variation de débit et/ou à au moins une durée de variation de débit prédéterminée

3. Dispositif selon la revendication 1 ou 2 ***caractérisé* en ce qu'il** comporte au moins un moyen de mesure de la surpression et/ou de la durée de la surpression lors de la déglutition et un moyen de comparaison automatique (30,31) de la surpression et/ou de la durée de la surpression par rapport à au moins une valeur de surpression et/ou à au moins une durée de surpression prédéterminée

4. Dispositif selon la revendication 3 ***caractérisé* en ce que** le moyen d'information indique l'écart entre la valeur de surpression et/ou la durée de surpression mesurée avec au moins une valeur de surpression et/ou au moins une durée de surpression prédéterminée.

5. Dispositif selon l'une quelconque des revendications 3 ou 4 ***caractérisé* en ce que** ledit moyen de comparaison automatique (30,31) compare la valeur de surpression et/ou la durée de surpression mesurée avec au moins une valeur de surpression et/ou à au moins une durée de surpression prédéterminée sur tout ou partie d'au moins l'une des trois phases de surpression appliquée dans le rhinopharynx lors de la déglutition, une phase de montée (100) de la surpression, une phase dite plateau (110) de la surpression pendant laquelle la surpression est sensiblement constante pendant une durée déterminée, et une phase de descente (120) de ladite surpression,

6. Dispositif selon la revendication 5 ***caractérisé* en ce que** la durée prédéterminée de la montée (100) en surpression est comprise entre 0,05 et 0,8 seconde.

7. Dispositif selon l'une quelconque des revendications 5 ou 6 ***caractérisé* en ce que** la durée prédéterminée de la phase plateau (110) de surpression est comprise entre 0,5 et 5 secondes.

8. Dispositif selon l'une quelconque des revendications 5 à 7 ***caractérisé* en ce que** la durée de la phase de descente (120) est comprise entre 0,05 et 0,8 seconde.

9. Dispositif selon l'une quelconque des revendications 3 à 8 ***caractérisé* en ce que** la valeur de surpression prédéterminée consiste en un pourcentage de la valeur de surpression mesurée à un instant prédéterminé de la phase plateau (110).

10. Dispositif selon l'une quelconque des revendications 3 à 9 ***caractérisé* en ce que** le moyen d'information (32) comporte des moyens d'alerte visuelle et/ou sonore.

11. Dispositif selon la revendication 10 ***caractérisé* en ce que** les moyens d'alerte visuelle consistent dans un écran (32) sur lequel sont aptes à s'afficher des messages.

12. Dispositif selon l'une quelconque des revendications 3 à 11 ***caractérisé* en ce que** le moyen de comparaison (30,31) comporte au moins un moyen de pilotage d'au moins un moyen de commande (35,37) apte à faire varier la valeur de la surpression mesurée.

13. Dispositif selon la revendication 12 ***caractérisé* en ce que** le moyen de commande consiste dans une électrovanne (35,37).

14. Dispositif selon l'une quelconque des revendications 3 à 13 ***caractérisé* en ce qu'il** comporte des moyens de transfert (34) des valeurs de surpression mesurées et/ou des valeurs des durées de surpression mesurées et/ou de la valeur de surpression prédéterminée et/ou de la valeur de la durée de surpression déterminée et/ou des écarts déterminés par le moyen de comparaison.

15. Dispositif selon l'une quelconque des revendications 1 à 14 ***caractérisé* en ce qu'il** comporte une soupape (40) dite de sécurité connectée à un tuyau flexible (5)
raccordant une buse d'arrivée d'air (4) de la pièce nasale (2) à une buse de sortie d'air (6) d'un boîtier (7) dans lequel s'étend le générateur de gaz (8) afin de limiter la pression dans le tuyau flexible (5) en dessous d'une pression déterminée.

## Patentansprüche

1. Vorrichtung zur automatischen Anwendung einer pneumatischen Überdruckphase mit einem zwischen 5 und 100 hPa inbegriffenen Wert im Gehörgang und / oder in den Nasenhöhlen eines menschlichen Patienten durch wenigstens ein Nasloch zum Zeitpunkt des Schluckens, wobei die genannte Vorrichtung wenigstens einen Gasgenerator (8), einen Gasbehälter (12) und einen Medienkreislauf zur Feststellung und Steuerung umfasst, der mit dem Behälter (12) und dem Gasgenerator (8) kommuniziert und bis zu einem abgedichteten Ansatzstück (1) an den Naslöchern reicht, das Bestandteil eines Nabenteils (2) ist, das mit einer Steuerdüse (3) ausgerüstet ist, **dadurch gekennzeichnet, dass** sie wenigstens ein Messmittel (29, 41) wenigstens eines charakteristischen Wertes eines Abfließens des Gases beim Schlucken, ein automatisches Vergleichsmittel (30, 31) des genannten charakteristischen Wertes mit wenigstens einem vorbestimmten Wert und ein Informationsmittel (32) des menschlichen Patienten, das geeignet ist anzuzeigen, ob das Schlucken ordnungsgemäß durchgeführt wurde, umfasst.

2. Vorrichtung gemäß Anspruch 1, **dadurch gekennzeichnet, dass** sie wenigstens ein Meßmittel der Durchsatzabweichung und / oder der Dauer der Durchsatzabweichung beim Schlucken und ein automatisches Vergleichsmittel des Wertes der Durchsatzabweichung und / oder der Dauer der Durchsatzabweichung im Verhältnis mit wenigstens einem Wert der Durchsatzabweichung und / oder wenigstens einer vorbestimmten Abweichungsdauer des Durchsatzes umfasst.

3. Vorrichtung gemäß Anspruch 1 oder 2, **dadurch gekennzeichnet, dass** sie wenigstens ein Meßmittel des Überdrucks und / oder der Dauer des Überdrucks beim Schlucken und ein automatisches Vergleichsmittel (30, 31) des Überdrucks und / oder der Dauer des Überdrucks im Verhältnis zu wenigstens einem Überdruckwert und / oder wenigstens einer vorbestimmten Überdruckdauer umfasst.

4. Vorrichtung gemäß Anspruch 3, **dadurch gekennzeichnet, dass** das Informationsmittel die Abweichung zwischen dem Überdruckwert und / oder der Überdruckdauer anzeigt, der gegenüber wenigstens einem Überdruckwert und / oder wenigstens einer vorbestimmten Überdruckdauer gemessen wurde.

5. Vorrichtung gemäß Anspruch 3 oder 4, **dadurch gekennzeichnet, dass** das genannte automatische Vergleichsmittel (30, 31) den gemessenen Überdruckwert und / oder die gemessene Überdruckdauer mit wenigstens einem vorbestimmten Überdruckwert und / oder wenigstens einer vorbestimmten Überdruckdauer über die ganze oder einen Teil wenigstens einer der drei Überdruckphasen, die auf den Nasenrachenraum beim Schlucken angewendet wird, einer Anstiegsphase (100) des Überdrucks, einer so genannten Plateauphase (110) des Überdrucks, während der der Überdruck während einer bestimmten Dauer deutlich konstant ist, und einer Abstiegsphase (120) des genannten Überdrucks vergleicht.

6. Vorrichtung gemäß Anspruch 5, **dadurch gekennzeichnet, dass** die vorbestimmte Dauer des Anstiegs (100) im Überdruck zwischen 0,05 und 0,8 Sekunden inbegriffen ist.

7. Vorrichtung gemäß Anspruch 5 oder 6, **dadurch gekennzeichnet, dass** die vorbestimmte Dauer der Plateauphase (110) des Überdrucks zwischen 0,5 und 5 Sekunden inbegriffen ist.

8. Vorrichtung gemäß Anspruch 5 bis 7, **dadurch gekennzeichnet, dass** die Dauer der Abstiegsphase (120) zwischen 0,05 und 0,8 Sekunden inbegriffen ist.

9. Vorrichtung gemäß Anspruch 3 bis 8, **dadurch gekennzeichnet, dass** der vorbestimmte Überdruckwert aus einem Prozentsatz des Überdruckwertes besteht, der zu einem vorbestimmten Moment der Plateauphase (110) gemessen wird.

10. Vorrichtung gemäß Anspruch 3 bis 9, **dadurch gekennzeichnet, dass** das Informationsmittel (32) visuelle und / oder akustische Warnmittel umfasst.

11. Vorrichtung gemäß Anspruch 10, **dadurch gekennzeichnet, dass** die visuellen Warnmittel aus einem Bildschirm (32) bestehen, auf dem Meldungen angezeigt werden können.

12. Vorrichtung gemäß Anspruch 3 bis 11, **dadurch gekennzeichnet, dass** das Vergleichsmittel (30, 31) wenigstens ein Lenkungsmittel wenigstens eines Steuermittels (35, 37) umfasst, das geeignet ist, den Wert des gemessenen Überdrucks schwanken zu lassen.

13. Vorrichtung gemäß Anspruch 12, **dadurch gekennzeichnet, dass** das Steuermittel aus einem Magnetventil (35, 37) besteht.

14. Vorrichtung gemäß Anspruch 3 bis 13, **dadurch gekennzeichnet, dass** sie Übertragungsmittel (34) der gemessenen Überdruckwerte und / oder der Werte der gemessenen Überdruckdauern und / oder des vorbestimmten Überdruckwerts und / oder des Werts der bestimmten Überdruckdauer und / oder durch das Vergleichsmittel bestimmte Abweichungen umfasst.

15. Vorrichtung gemäß Anspruch 1 bis 14, **dadurch gekennzeichnet, dass** sie ein so genanntes Sicherheitsventil (40) umfasst, das an einen flexiblen Schlauch (5) angeschlossen ist, der eine Lufteingangsdüse (4) des Nabenteils (2) mit einer Luftausgangsdüse (6) eines Gehäuses (7) verbindet, in dem sich der Gasgenerator (8) erstreckt, um den Druck in dem flexiblen Schlauch (5) unterhalb eines bestimmten Drucks zu begrenzen.

## Claims

1. Device for automatically applying a pneumatic overpressure phase, of a value of between 5 and 100 hPa in the Eustachian tube and/or in the nasal fossae of a human being through at least one nostril at the moment of swallowing, the said device comprising at least one gas generator (8), one gas reservoir (12) and one detection-fluid and control circuit communicating with the gas reservoir (12) and gas generator (8) and leading to a sealed plug (1) in the nostrils forming part of a nosepiece (2) equipped with a control nozzle (3); ***characterised* in that** it comprises at least one means of measuring (29,41) at least one characteristic value of a flow of gas during swallowing, one means of automatically comparing (30, 31) the said characteristic value with at least one pre-set value, and one means of informing (32) the patient capable of indicating whether the swallowing action is being correctly performed.

2. Device according to claim 1 ***characterised* in that** it comprises at least one means of measuring the variation of the flow and/or the duration of the variation of the flow on swallowing and one means of automatically comparing the value of the variation of the flow and/or the duration of the variation of the flow with at least one pre-set value of variation of flow and/or with at least one pre-set duration of variation of flow.

3. Device according to claims 1 or 2 ***characterised* in that** it comprises at least one means of measuring the overpressure and/or the overpressure duration on swallowing and one means of automatically comparing (30,31) the overpressure and/or the overpressure duration with at least one pre-set value of overpressure and/or at least one pre-set duration of overpressure.

4. Device according to claim 3 ***characterised* in that** the information means indicate the difference between the overpressure value and/or the overpressure duration measured and at least one pre-set overpressure value and/or at least one pre-set overpressure duration.

5. Device according to either of claims 3 or 4 ***characterised* in that** the said means of automatic comparison (30,31) compare the measured overpressure value and/or the measured duration of overpressure with at least one pre-set overpressure value and/or with at least one pre-set duration of overpressure over all or part of at least one of the three overpressure phases applied in the nasopharynx during swallowing, one overpressure increase phase (100), one so-called overpressure plateau phase (110) during which the overpressure is substantially constant for a set duration, and one decrease phase (120) of the said overpressure.

6. Device according to either of claims 5 or 6 ***characterised* in that** the pre-set duration of the increase (100) in overpressure is within 0.05 and 0.8 of a second.

7. Device according to either of claims 5 or 6 ***characterised* in that** the pre-set duration of the overpressure plateau phase (110) is within 0.5 and 5 seconds.

8. Device according to any of claims 5 to 7 ***characterised* in that** the duration of the decrease phase (120) is within 0.05 and 0.8 of a second.

9. Device according to any of claims 3 to 8 ***characterised* in that** the pre-set overpressure value consists in a percentage of the overpressure value measured at a pre-set moment of the plateau phase (110).

10. Device according to any of claims 3 to 9 ***characterised* in that** the information means (32) comprise visual and/or audible warning means.

11. Device according to claim 10 ***characterised* in that** the visual warning means consist in a screen (32) on which messages can be displayed.

12. Device according to any of claims 3 to 11 ***characterised* in that** the comparison means (30,31) comprise at least one monitoring means of at least one control means (35,37) capable of altering the value of the measured overpressure.

13. Device according to claim 12 ***characterised* in that** the control means consist in an electrovalve (35,37).

14. Device according to any of claims 3 to 13 ***characterised* in that** it comprises means of transferring (34) the measured overpressure values and/or the measured overpressure duration values and/or the pre-set overpressure value and/or the set overpressure duration value and/or the differences determined by the comparison means.

15. Device according to any of claims 1 to 14 ***characterised* in that** it comprises a so-called safety valve (40) connected to a flexible pipe (5) connecting an air inlet nozzle (4) of the nosepiece (2) to an air outlet nozzle (6) of a case (7) containing a gas generator (8) in order to limit the pressure in the flexible pipe (5) to below a set pressure.
